# EUROPEAN PATENT APPLICATION

(11) **EP 4 840 847 A1**
(43) Date of publication of application: **18.11.2026**
(21) Application number: 25738950.2
(22) Date of filing: 08.01.2025
(51) Int. Cl.: C07D 207/27, C07D 207/267

(54) **METHOD FOR PREPARING N-VINYL-2-PYRROLIDONE INTERMEDIATE AND N-VINYL-2-PYRROLIDONE INTERMEDIATE PREPARED THEREFROM**

(30) Priority: 09.01.2024 KR 20240003513
(71) Applicant: CJ Cheiljedang Corporation, Seoul 04560 (KR)
(72) Inventor: YOU, Youngsu, Seoul 04560 (KR); SHIN, Hun Yi, Seoul 04560 (KR); SEO, Jang-Woo, Seoul 04560 (KR); KIM, Tae Jun, Seoul 04560 (KR); PARK, Jeung Yil, Seoul 04560 (KR); JEON, Jinwoo, Seoul 04560 (KR)
(74) Representative: Meissner Bolte Partnerschaft mbB
(86) International application number: PCT/KR2025/000410
(87) International publication number: WO 2025/150882

(57) **Abstract**

The present invention relates to a method for preparing an N-vinyl-2-pyrrolidone intermediate, to an N-vinyl-2-pyrrolidone intermediate prepared by the preparation method, and to N-vinyl-2-pyrrolidone prepared from the N-vinyl-2-pyrrolidone intermediate. The preparation method comprises the steps of: (1) preparing a reactant containing a polyhydroxyalkanoate obtained by microbial cultivation; and (2) reacting the reactant containing the polyhydroxyalkanoate with an amine compound at a temperature exceeding 200 °C so as to produce an intermediate-containing product.

## Description

### Technical Field

The present disclosure relates to a process for preparing an intermediate (precursor) used in the preparation of N-vinyl-2-pyrrolidone (NVP), which is a high-value-added chemical, in a high yield.

### Background Art

Bio-refinery is a technology for preparing biofuel (energy), power, heat, and high-value-added chemicals from biomass through biological or chemical conversion processes.

In recent years, technologies have been developed for chemically depolymerizing polyhydroxyalkanoate (PHA), one of the above biomass materials, to produce high-value-added chemicals or intermediates. For example, an N-vinyl-2-pyrrolidone (NVP) intermediate or N-vinyl-2-pyrrolidone (NVP) may be prepared using polyhydroxyalkanoate.

The NVP intermediate is a raw material used in the preparation of NVP, which is a high-value-added chemical. An improvement in its yield can lead to an enhancement in the productivity of NVP.

Conventionally, NVP intermediates or NVP have been prepared through a procedure in which 1,4-butanediol (1,4-BDO) is obtained from petroleum-derived acetylene, it is converted to γ-butyrolactone (GBL), and then 2-pyrrolidone, N-vinyl-2-pyrrolidone (NVP), and the like are produced. However, this preparation process has a long reaction step and increases the unit cost of NVP due to the use of expensive metal catalysts.

Meanwhile, the preparation of NVP intermediates or NVP using polyhydroxyalkanoate also has long and complex reaction steps, and the yields of NVP intermediates and NVP do not reach satisfactory levels.

### Disclosure of Invention

### Technical Problem

As described above, conventionally, there have been limitations in producing NVP intermediates or NVP in an environmentally friendly and economically feasible manner in a high yield. In particular, when NVP intermediates are prepared using polyhydroxyalkanoate, which is a biomass, environmental problems and the limitation of petroleum resource depletion can be solved, whereas it has the problem of long and complex reaction steps and a significantly reduced yield of NVP intermediates.

The present inventors have conducted various studies to solve the above problems. As a result, it has been discovered that N-vinyl-2-pyrrolidone (NVP) intermediates can be prepared in a high yield through relatively simple reaction steps by controlling the composition of a reactant containing polyhydroxyalkanoate and the reaction temperature of the reactant.

Therefore, an object of the present disclosure is to provide a process for preparing an N-vinyl-2-pyrrolidone (NVP) intermediate capable of producing the N-vinyl-2-pyrrolidone (NVP) intermediate in a high yield through simple reaction steps while using a reactant containing polyhydroxyalkanoate.

In addition, another object of the present disclosure is to provide an N-vinyl-2-pyrrolidone (NVP) intermediate prepared by the above preparation process and N-vinyl-2-pyrrolidone (NVP) prepared using the same.

### Solution to Problem

In order to accomplish the above object, the present disclosure provides a process for preparing an N-vinyl-2-pyrrolidone intermediate that comprises: (1) preparing a reactant containing polyhydroxyalkanoate obtained through microbial cultivation; and (2) reacting the reactant containing the polyhydroxyalkanoate with an amine compound at a temperature exceeding 200°C to produce an intermediate-containing product.

According to an embodiment of the present disclosure, in step (1), the polyhydroxyalkanoate may comprise a repeat unit derived from 4-hydroxybutyrate.

According to another embodiment of the present disclosure, in step (1), the content of the polyhydroxyalkanoate contained in the reactant may be 60% by weight to 90% by weight based on the total weight of the reactant.

According to another embodiment of the present disclosure, in step (1), the moisture content of the reactant may be 0% by weight to 80% by weight based on the total weight of the reactant.

According to another embodiment of the present disclosure, in step (1), the reactant may further comprise an inorganic substance.

According to another embodiment of the present disclosure, the content of the inorganic substance may be greater than 0% by weight to 10% by weight based on the total weight of the reactant.

According to another embodiment of the present disclosure, the inorganic substance may comprise at least one selected from the group consisting of calcium carbonate (CaCO₃), ferric sulfate (Fe₂(SO₄)₃), and sodium hydrogen carbonate (NaHCO₃).

According to another embodiment of the present disclosure, in step (2), the initial reaction pressure of the reactant and the amine compound may be 1 bar to 120 bar.

According to another embodiment of the present disclosure, in step (2), the amine compound may comprise a compound selected from the group consisting of monoethanolamine, an aqueous monoethanolamine solution, ammonia, aqueous ammonia, urea, and aqueous urea.

According to another embodiment of the present disclosure, in step (2), the reactant and the amine compound may react at an equivalent ratio of 1:1.1 to 3.

According to another embodiment of the present disclosure, in step (2), the intermediate may be N-(2-hydroxyethyl)-2-pyrrolidone.

Meanwhile, the present disclosure provides an N-vinyl-2-pyrrolidone intermediate prepared by the process for preparing an N-vinyl-2-pyrrolidone intermediate.

In addition, the present disclosure provides N-vinyl-2-pyrrolidone prepared from the N-vinyl-2-pyrrolidone intermediate.

In addition, the present disclosure provides a process for preparing N-vinyl-2-pyrrolidone, which comprises: reacting an N-vinyl-2-pyrrolidone intermediate prepared by the process for preparing an N-vinyl-2-pyrrolidone intermediate with acetic anhydride to obtain an acetic ester intermediate; and deacetylating the obtained acetic ester intermediate.

In addition, the present disclosure provides a process for preparing N-vinyl-2-pyrrolidone, which comprises: subjecting an N-vinyl-2-pyrrolidone intermediate prepared by the process for preparing an N-vinyl-2-pyrrolidone intermediate to intramolecular dehydration in a vapor phase in the presence of a catalyst.

### Advantageous Effects of Invention

The present disclosure can prepare an N-vinyl-2-pyrrolidone intermediate (N-vinyl-2-pyrrolidone precursor) in a high yield and at a low cost by producing it through relatively simple (short) reaction steps, as compared with the prior art, without using a metal catalyst.

Furthermore, since the yield of the N-vinyl-2-pyrrolidone intermediate is high in the present disclosure, it is possible to increase the production rate of N-vinyl-2-pyrrolidone prepared using the N-vinyl-2-pyrrolidone intermediate. As a result, it can contribute to providing N-vinyl-2-pyrrolidone, which is a synthetic raw material for polyvinylpyrrolidone (PVP) widely used in the manufacture of cosmetics, foods, pharmaceuticals, paper, textiles, pigments, paints, electronic devices, and the like, with high efficiency.

### Brief Description of Drawings

Fig. 1 is a flowchart showing the preparation process of an N-vinyl-2-pyrrolidone intermediate according to an embodiment of the present disclosure.
Fig. 2 is a graph showing the results according to Test Example 1.

### Best Mode for Carrying out the Invention

Hereinafter, the present disclosure will be described in detail. The present disclosure is not limited to those given below, but it may be modified into various forms as long as the gist of the disclosure is not changed.

In the present specification, the term "comprising" is intended to specify a particular characteristic, region, step, process, element, and/or component. It does not exclude the presence or addition of any other characteristic, region, step, process, element and/or component, unless specifically stated to the contrary.

All numbers and expressions related to the quantities of components, reaction conditions, and the like used herein are to be understood as being modified by the term "about" unless otherwise indicated.

The present disclosure provides a process for preparing an N-vinyl-2-pyrrolidone intermediate using polyhydroxyalkanoate, which is a biomass, an N-vinyl-2-pyrrolidone intermediate prepared by the process, N-vinyl-2-pyrrolidone prepared from the N-vinyl-2-pyrrolidone intermediate, and a process for preparing the same. The present disclosure has the characteristic that an N-vinyl-2-pyrrolidone intermediate can be prepared in a high yield through simple reaction steps without using expensive metal catalysts by optimizing the composition of a reactant containing polyhydroxyalkanoate (e.g., the components of the reactant, the moisture content of the reactant, etc.) and the reaction temperature of the reactant, which will be described in detail, as follows.

### Process for preparing an N-vinyl-2-pyrrolidone Intermediate

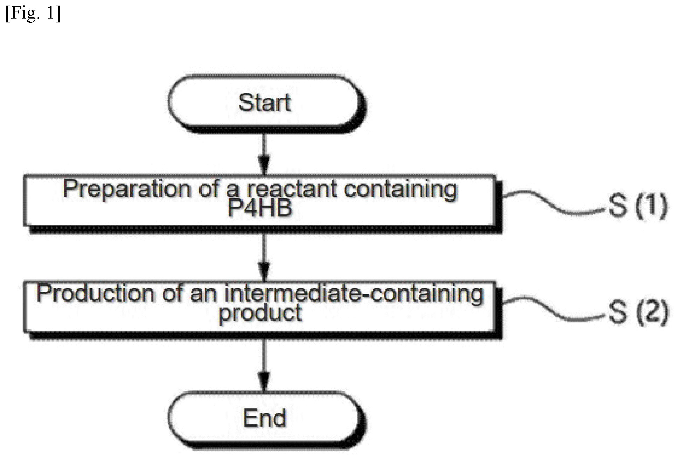

The process for preparing an N-vinyl-2-pyrrolidone intermediate (hereinafter referred to as "NVP intermediate") according to the present disclosure comprises: (1) preparing a reactant containing polyhydroxyalkanoate obtained through microbial cultivation; and (2) reacting the reactant containing the polyhydroxyalkanoate with an amine compound at a temperature exceeding 200°C to produce an intermediate-containing product, which will be described with reference to Fig. 1, as follows.

### Step (1): Preparation of a reactant

According to the present disclosure, step (1) is a step of preparing a reactant containing polyhydroxyalkanoate obtained through microbial cultivation (S (1)).

The reactant is not particularly limited as long as it is a material containing polyhydroxyalkanoate. Specifically, the reactant is a material obtained from a cell fermentation broth containing polyhydroxyalkanoate, and it may comprise polyhydroxyalkanoate, cells, cell-derived materials, and debris. In addition, the reactant may be polyhydroxyalkanoate itself obtained through a procedure of separating and purifying the material obtained from a cell fermentation broth by a conventional method.

For example, the reactant may be a dried cell material obtained by adding a polymer flocculant and water (e.g., distilled water) to a cell fermentation broth containing polyhydroxyalkanoate to obtain a cell flocculation liquid, and dehydrating the obtained cell flocculation liquid. In addition, the reactant may be a cell fermentation broth that has not undergone the flocculation and dehydration, or it may be a cell flocculation liquid (or cell flocculate) that has undergone the flocculation, but not the dehydration. The cell fermentation broth or the cell flocculation liquid (or cell flocculate) has not undergone a purification process (e.g., dehydration process). As it is applied as a reactant, the preparation process of an NVP intermediate can be shortened while reducing the cost consumed in the purification process. As a result, it is possible to prepare an NVP intermediate and/or NVP in an economically feasible manner.

The cell fermentation broth may be obtained by culturing microorganisms having the ability to produce biomass containing polyhydroxyalkanoate. The microorganism may comprise one or more selected from the group consisting of *Bacillus subtilis*, *Cupriavidus necator*, *Bacillus cereus*, *Bacillus brevis*, *Caulobacter cresentus*, *Bacillus sphaericus*, *Bacillus coagulans*, *Bacillus megaterium*, *Bacilllus circulands*, *Bacillus licheniformis*, *Escherichia coli*, *Microlanatus phosphovorous*, *Rhizobium meliloti*, *Rhizobium viciae*, *Bredyrhizobium japonicum*, *Burkholderia cepacia*, *Burkholderia sacchari*, *Cupriavidus necactor*, *Neptunamonas Antarctica*, *Azobacter vinelandii*, *Pseudomonas putida*, *Pseudomonas aeruginosa*, *Aeromonas caviae*, *Aeromonas hydrophila*, *Aeromonas punctate*, *Alcaligenes latus*, *Halomonas boliviensis*, *Lactobacillus rhamnosus*, and *Fermicutes bacterium*, but it is not limited thereto.

The polymer flocculant is not particularly limited as long as it is a polymer material capable of flocculating the cell fermentation broth. Specifically, the polymer flocculant may comprise a cationic polymer flocculant having a molecular weight of 1,000,000 Da or more (e.g., 1,000,000 Da to 10,000,000 Da, 1,000,000 Da to 5,000,000 Da, 1,300,000 Da to 4,500,000 Da, 1,500,000 Da to 4,000,000 Da, 2,000,000 Da to 3,500,000 Da, 2,300,000 Da to 3,000,000 Da, or 2,500,000 Da to 3,000,000 Da). For example, the polymer flocculant may be a polymer flocculant solution in which the cationic polymer flocculant is dissolved or dispersed in a solvent (e.g., distilled water). As the polymer flocculant comprises the cationic polymer flocculant, the flocculation efficiency of the cell fermentation broth containing polyhydroxyalkanoate, which is anionic, can be significantly increased.

For example, the cationic polymer flocculant may be a flocculant comprising an acrylamide-based polymer, an acryloyl-based polymer, an amidine-based polymer, a polyvinylamine-based polymer, a diallyldimethylammonium chloride-based polymer, a polyamine-based polymer, or a combination thereof. Specifically, it may be a polyacrylamide-based polymer flocculant.

The dehydration of the cell flocculation liquid may be performed using at least one of a press and an electroosmotic dehydrator, but it is not limited thereto. Specifically, the cell flocculation liquid may be primarily dehydrated with a press to obtain a flocculate, which is then secondarily dehydrated with the electroosmotic dehydrator to obtain a reactant (dried cell material). Through such dehydration, the present disclosure can minimize the content of impurities contained in the reactant while controlling the moisture content of the reactant as desired.

The press is not particularly limited as long as it is a commonly known press. Specifically, it may be a screw press, a wedge press, a hydraulic press, or the like.

The operating pressure of the press (e.g., the operating pressure of the press plate provided in the press) is not particularly limited, but it may be 0.01 MPa to 1 MPa. Specifically, the operating pressure of the press may be 0.03 MPa to 0.9 MPa, 0.05 MPa to 0.7 MPa, 0.1 MPa to 0.6 MPa, 0.2 MPa to 0.5 MPa, or 0.3 MPa to 0.4 MPa, but it is not limited thereto. As the operating pressure of the press is within the above range, primary dehydration can be efficiently performed while preventing damage to the polyhydroxyalkanoate.

The electroosmotic dehydrator may not be particularly limited as long as it is a dehydrator that applies the electrophoresis principle.

The operating conditions of the electroosmotic dehydrator are not particularly limited, but the operating speed may be 0.1 m/minute to 4 m/minute, and the applied voltage may be 10 V to 150 V. Specifically, the operating speed of the electroosmotic dehydrator may be 0.5 m/minute to 3.5 m/minute, 0.7 m/minute to 3 m/minute, 0.9 m/minute to 2.5 m/minute, 1 m/minute to 2 m/minute, or 1.1 m/minute to 1.5 m/minute, and the applied voltage of the electroosmotic dehydrator may be 12 V to 130 V, 13 V to 100 V, 15 V to 50 V, 17 V to 30 V, or 19 V to 25 V, but they are not limited thereto. As the operating conditions of the electroosmotic dehydrator are within the above ranges, the secondary dehydration and impurity removal can be efficiently performed.

Meanwhile, in order to promote the fluidity and chemical reactivity of the cell flocculation liquid, an inorganic substance may be further added to the cell fermentation broth together with the polymer flocculant. As a result, the reactant obtained through the flocculation and/or dehydration may further comprise an inorganic substance.

According to the present disclosure, the content of the inorganic substance further contained in the reactant may be greater than 0% by weight to 10% by weight based on the total weight of the reactant (e.g., a reactant having a moisture content of 0% by weight). Specifically, the content of the inorganic substance in the reactant may be 0.1% by weight to 9.8% by weight, 0.3% by weight to 9.5% by weight, 0.5% by weight to 9% by weight, 1% by weight to 8% by weight, 1.5% by weight to 7% by weight, 2% by weight to 6.5% by weight, 2.5% by weight to 6% by weight, 3% by weight to 5.5% by weight, 3.5% by weight to 5% by weight, or 4% by weight to 5% by weight, based on the total weight of the reactant, but it is not limited thereto. As the content of the inorganic substance in the reactant is within the above range, the fluidity and chemical reactivity of the reactant can be optimized.

When the fluidity and chemical reactivity of the reactant are considered, the inorganic substance may comprise at least one selected from the group consisting of calcium carbonate (CaCO₃), ferric sulfate (Fe₂(SO₄)₃), and sodium hydrogen carbonate (NaHCO₃), but it is not limited thereto. For example, the inorganic substance may comprise all of calcium carbonate (CaCO₃), ferric sulfate (Fe₂(SO₄)₃), and sodium hydrogen carbonate (NaHCO₃).

According to the present disclosure, the moisture content of the reactant may be controlled to increase the yield of the NVP intermediate. For example, the reactant obtained through the flocculation and dehydration may have a moisture content of 0% by weight, and the moisture content of the reactant may be controlled by adding water (e.g., distilled water) to the reactant having a moisture content of 0% by weight. In addition, the reactant is a cell fermentation broth that has not undergone the flocculation and dehydration, and it may have a controlled moisture content by comprising moisture derived from the cell fermentation broth. In addition, the reactant is a cell flocculation liquid (or cell flocculate) that has undergone the flocculation but not the dehydration, and it may have a controlled moisture content by comprising moisture derived from the cell fermentation broth and the cell flocculation liquid (or cell flocculate).

Specifically, the content of moisture contained in the reactant may be 0% by weight to 80% by weight based on the total weight of the reactant. More specifically, the moisture content of the reactant may be 5% by weight to 80% by weight, 10% by weight to 79% by weight, 15% by weight to 78% by weight, 20% by weight to 77% by weight, 25% by weight to 76% by weight, 30% by weight to 75% by weight, 35% by weight to 74% by weight, 40% by weight to 73% by weight, 45% by weight to 72% by weight, 50% by weight to 71% by weight, or 55% by weight to 70% by weight, based on the total weight of the reactant, but it is not limited thereto. As the moisture content of the reactant is within the above range, the conversion rate of polyhydroxyalkanoate can be maximized, thereby significantly increasing the yield of the NVP intermediate.

Meanwhile, the polyhydroxyalkanoate (PHA) contained in the reactant is a thermoplastic natural polyester polymer accumulated in the cells of microorganisms. It has physical properties similar to those of synthetic biodegradable polymers derived from petroleum, such as polybutylene adipate terephthalate (PBAT), polybutylene succinate (PBS), polybutylene succinate terephthalate (PBST), and polybutylene succinate adipate (PBSA), and has excellent characteristics in terms of biodegradability and biocompatibility.

The polyhydroxyalkanoate may have a weight average molecular weight (Mw) of 100,000 g/mole to 1,000,000 g/mole, 200,000 g/mole to 900,000 g/mole, 300,000 g/mole to 800,000 g/mole, 400,000 g/mole to 700,000 g/mole, or 500,000 g/mole to 600,000 g/mole, but it is not limited thereto.

The polyhydroxyalkanoate may comprise a repeat unit derived from (from one or more monomers) at least one selected from the group consisting of 3-hydroxybutyrate (3HB), 3-hydroxypropionate (3HP), 3-hydroxyhexanoate (3HH), 3-hydroxyvalerate (3HV), 4-hydroxybutyrate (4HB), 4-hydroxyvalerate (4HV), 5-hydroxyvalerate (5HV), and 6-hydroxyhexanoate (6HH).

Specifically, the polyhydroxyalkanoate may be a homopolymer comprising a repeat unit derived from 3-hydroxybutyrate (3HB) or a repeat unit derived from 4-hydroxybutyrate (4HB). In addition, the polyhydroxyalkanoate may be a copolymer comprising a repeat unit derived from 3-hydroxybutyrate (3HB) and a repeat unit derived from 4-hydroxybutyrate (4HB).

Preferably, the polyhydroxyalkanoate may be a homopolymer comprising a repeat unit derived from 4-hydroxybutyrate (4HB) (e.g., poly-4-hydroxybutyrate (P4HB)). As the polyhydroxyalkanoate comprises a repeat unit derived from 4-hydroxybutyrate (4HB), the reaction with an amine compound described below at high temperatures is optimized, thereby increasing the yield of the NVP intermediate.

According to the present disclosure, the content of the polyhydroxyalkanoate contained in the reactant may be 60% by weight to 90% by weight based on the total weight of the reactant (e.g., a reactant having a moisture content of 0% by weight). Specifically, the content of the polyhydroxyalkanoate in the reactant may be 63% by weight to 90% by weight, 65% by weight to 89% by weight, 68% by weight to 88% by weight, 70% by weight to 86% by weight, 73% by weight to 85% by weight, 75% by weight to 83% by weight, 76% by weight to 82% by weight, or 78% by weight to 80% by weight, based on the total weight of the reactant, but it is not limited thereto. As the content of the polyhydroxyalkanoate in the reactant is within the above range, the yield of the NVP intermediate can be increased.

### Step (2): Production of an intermediate-containing product

According to the present disclosure, step (2) is a step of reacting the reactant containing the polyhydroxyalkanoate with an amine compound at a temperature exceeding 200°C to produce an intermediate-containing product (S (2)).

The reaction temperature of the reactant and the amine compound is not particularly limited as long as it is a temperature exceeding 200°C. Specifically, the reaction temperature may be 205°C or higher, 210°C or higher, 215°C or higher, 220°C or higher, 225°C or higher, 230°C or higher, 235°C or higher, 240°C or higher, 245°C or higher, or 250°C or higher, and may be 350°C or lower, 340°C or lower, 330°C or lower, 320°C or lower, 310°C or lower, 300°C or lower, 290°C or lower, 280°C or lower, 270°C or lower, 260°C or lower, or 250°C or lower, but it is not limited thereto. For example, the reaction temperature may be 210°C to 350°C, 220°C to 330°C, 230°C to 310°C, 240°C to 290°C, or 250°C to 270°C.

According to the present disclosure, the initial reaction pressure (initial set pressure for reaction progression) of the reactant and the amine compound may be 1 bar to 120 bar. Specifically, the initial reaction pressure may be 1 bar to 115 bar, 3 bar to 110 bar, 5 bar to 105 bar, 7 bar to 100 bar, 10 bar to 90 bar, 15 bar to 85 bar, 20 bar to 80 bar, 25 bar to 75 bar, 30 bar to 70 bar, 35 bar to 65 bar, 40 bar to 60 bar, or 50 bar to 55 bar, but it is not limited thereto.

As the reaction temperature and the initial reaction pressure are respectively within the above ranges, the conversion rate at which the polyhydroxyalkanoate contained in the reactant is converted to an intermediate (e.g., N-(2-hydroxyethyl)-2-pyrrolidone) desired in the present disclosure can be significantly increased. As a result, an NVP intermediate can be prepared in a high yield through simple reaction steps.

Meanwhile, the pressure during the reaction measured after the reaction of the reactant and the amine compound has been carried out for 30 minutes is not particularly limited, but it may be 10 bar to 150 bar, 15 bar to 140 bar, 18 bar to 130 bar, 20 bar to 120 bar, 25 bar to 110 bar, 30 bar to 100 bar, 40 bar to 90 bar, or 50 bar to 80 bar.

In addition, the reaction time of the reactant and the amine compound is not particularly limited. However, in light of the yield of the NVP intermediate, it may be 1 hour to 10 hours, 1.5 hours to 8 hours, 2 hours to 6 hours, 2.5 hours to 5 hours, or 3 hours to 4 hours.

According to the present disclosure, the reaction ratio of the reactant and the amine compound may be an equivalent ratio of 1:1.1 to 3. Specifically, in light of the yield of the NVP intermediate, the reaction ratio may be an equivalent ratio of 1:1.3 to 3, an equivalent ratio of 1:1.5 to 2.8, an equivalent ratio of 1:1.8 to 2.5, or an equivalent ratio of 1:1.9 to 2.2, but it is not limited thereto.

The amine compound is not particularly limited as long as it is a compound capable of depolymerizing the polyhydroxyalkanoate contained in the reactant. Specifically, the amine compound may comprise a compound selected from the group consisting of monoethanolamine, an aqueous monoethanolamine solution, ammonia, aqueous ammonia, urea, and aqueous urea. As the amine compound comprises the above compound, the conversion rate of the polyhydroxyalkanoate is increased, thereby enhancing the yield of the NVP intermediate.

Meanwhile, according to the present disclosure, the intermediate contained in the intermediate-containing product is not particularly limited, but it may be N-(2-hydroxyethyl)-2-pyrrolidone. As the intermediate is N-(2-hydroxyethyl)-2-pyrrolidone (HEP), the reaction steps for obtaining the NVP intermediate are minimized, thereby increasing the production efficiency of the NVP intermediate. Specifically, N-(2-hydroxyethyl)-2-pyrrolidone may be the NVP intermediate, which is the target product in the present disclosure.

The intermediate-containing product may comprise, in addition to the intermediate, by-products such as γ-butyrolactone (GBL), 2-pyrrolidone, N-methylpyrrolidone (NMP), N-vinyl-2-pyrrolidone (NVP), 4-hydroxybutyric acid (4HB), and 4-hydroxy-N-(2-hydroxyethyl)butanamide (4HEBA).

In particular, the intermediate-containing product may comprise N-(2-hydroxyethyl)-2-pyrrolidone in a high content and may comprise 4-hydroxy-N-(2-hydroxyethyl)butanamide in a low content. For example, the content of N-(2-hydroxyethyl)-2-pyrrolidone contained in the intermediate-containing product may be 10% by weight to 99% by weight, 20% by weight to 90% by weight, 30% by weight to 80% by weight, 40% by weight to 70% by weight, or 50% by weight to 60% by weight, based on the total weight of the intermediate-containing product, but it is not limited thereto. In addition, the content of 4-hydroxy-N-(2-hydroxyethyl)butanamide contained in the intermediate-containing product may be 0% by weight to 15% by weight, 0.2% by weight to 12% by weight, 0.6% by weight to 9% by weight, 1% by weight to 6% by weight, 1.4% by weight to 3% by weight, or 2% by weight to 2.5% by weight, based on the total weight of the intermediate-containing product, but it is not limited thereto.

According to the present disclosure, after the production of the intermediate-containing product through steps (1) and (2), the NVP intermediate can be obtained through conventional filtering and extraction procedures.

As described above, the present disclosure prepares the N-vinyl-2-pyrrolidone intermediate through simple reaction steps of steps (1) and (2) without using expensive metal catalysts; thus, it can achieve a high yield while reducing the cost consumed in the preparation of the N-vinyl-2-pyrrolidone intermediate.

According to the present disclosure, the yield of the N-vinyl-2-pyrrolidone intermediate (e.g., N-(2-hydroxyethyl)-2-pyrrolidone) may be 35% or more, 38% or more, 40% or more, 43% or more, 45% or more, 50% or more, 53% or more, 55% or more, 60% or more, 65% or more, 70% or more, 75% or more, 80% or more, or 85% or more (e.g., 35% to 95%, 40% to 93%, 50% to 90%, or 60% to 88%), but it is not limited thereto.

### N-vinyl-2-pyrrolidone Intermediate

The N-vinyl-2-pyrrolidone intermediate (precursor) according to the present disclosure is prepared by the preparation process described above. The N-vinyl-2-pyrrolidone intermediate exhibits a high yield and a high purity as it is prepared by the preparation process described above, and it can be advantageously used in the preparation of N-vinyl-2-pyrrolidone, which is a high-value-added chemical.

### N-vinyl-2-pyrrolidone and process for preparing the same

N-vinyl-2-pyrrolidone according to the present disclosure is prepared from the N-vinyl-2-pyrrolidone intermediate described above. The preparation of N-vinyl-2-pyrrolidone using the N-vinyl-2-pyrrolidone intermediate can be performed by a conventionally known preparation process.

For example, the process for preparing the N-vinyl-2-pyrrolidone may comprise: reacting an N-vinyl-2-pyrrolidone intermediate prepared by the process for preparing an N-vinyl-2-pyrrolidone intermediate with acetic anhydride to obtain an acetic ester intermediate; and deacetylating the obtained acetic ester intermediate, but it is not limited thereto. The reaction with acetic anhydride and the deacetylation may have reaction conditions set in light of the yield of N-vinyl-2-pyrrolidone.

In addition, the process for preparing N-vinyl-2-pyrrolidone may comprise subjecting an N-vinyl-2-pyrrolidone intermediate prepared by the preparation process described above to intramolecular dehydration in a vapor phase in the presence of a catalyst, but it is not limited thereto. The dehydration may have reaction conditions set in light of the yield of N-vinyl-2-pyrrolidone.

The present disclosure can prepare the N-vinyl-2-pyrrolidone intermediate in a high yield, thereby increasing the production amount of N-vinyl-2-pyrrolidone, thereby reducing the unit cost of N-vinyl-2-pyrrolidone.

N-vinyl-2-pyrrolidone can be advantageously used as a synthetic raw material (monomer) for polyvinylpyrrolidone (PVP) widely used in the manufacture of cosmetics, foods, pharmaceuticals, paper, textiles, pigments, paints, electronic devices, and the like.

### Mode for the Invention

Hereinafter, the present disclosure will be described in detail with reference to Examples, but the scope of the present disclosure is not limited to the Examples.

### [Reagents, test equipment, and analysis equipment]

In the examples, comparative examples, and test examples, the following reagents were used: isopropyl alcohol (Daejeong Chemical, HPLC grade), monoethanolamine (TCI, >99.0%), acetonitrile (BURDICK&JACKSON, HPLC grade), distilled water (MALLINCKRODT BAKER, HPLC grade), cationic polymer flocculant (FO4800VHM, SNF Korea), calcium carbonate (CaCO₃) (Sigma Aldrich, >99%), ferric sulfate (Fe₂(SO₄)₃) (Chemtech International, iron content 10.5% or more), and sodium hydrogen carbonate (NaHCO₃) (Sigma Aldrich, >99%).

The following test equipment was used: seal tube (CHEMGLASS LIFE SCIENCES, P05-132-128(1880-04)), high-temperature and high-pressure reactor (Hanwool Engineering, Reactor system), heating magnetic stirrer (Daehan Science, DH.WMH03021), press (Hwain, Screw press dehydrator SP-30), and electroosmotic dehydrator (Hwain, EOD-500S).

High-performance liquid chromatography (HPLC) and gas chromatography were used as analysis equipment. Specifically, 1260 Infinity equipment from Agilent Technologies was used as the HPLC equipment, and Capcell Pak C18 MG (4.6 mm × 250 mm × 5 µm, P/N 92635) from Osaka Soda was used as the column. Here, the autosampler temperature was set at 15°C, the column temperature was set at 35°C, tertiary distilled water containing 0.2% phosphoric acid and acetonitrile (ACN) was used as a mobile phase solvent, and the concentration was changed (gradient) to carry out analysis at a mobile phase speed of 1 ml/minute. GC (Agilent Technologies, 8890) equipped with a DB-WAX (60 m × 250 µm × 0.25 µm) column was used as the GC equipment. Here, the inlet temperature was set at 250°C, and the flow rate was 1 ml/minute. In addition, the flame ionization detector (FID) temperature was set at 300°C for the analysis. Meanwhile, when products were analyzed, GC samples were diluted with isopropyl alcohol (IPA), and HPLC samples were diluted with distilled water before analysis.

### [Example 1]

### Preparation of a reactant containing poly-4-hydroxybutyrate (P4HB)

A cationic polymer flocculant and distilled water were added to a cell fermentation broth containing P4HB for the flocculation thereof. Subsequently, calcium carbonate (CaCO₃), ferric sulfate (Fe₂(SO₄)₃), and sodium hydrogen carbonate (NaHCO₃) for promoting flow and chemical reaction were added at a certain ratio to the cell flocculation liquid in which the cell fermentation broth was flocculated. Next, the cell flocculation liquid was dehydrated with a press and an electroosmotic dehydrator to obtain a reactant containing P4HB. The content of P4HB contained in the obtained reactant was about 78.3% by weight, the total content of CaCO₃, Fe₂(SO₄)₃, and NaHCO₃ added was about 5% by weight, the moisture content was 0% by weight, and the balance was cell debris.

### Preparation of a product containing N-(2-hydroxyethyl)-2-pyrrolidone (HEP)

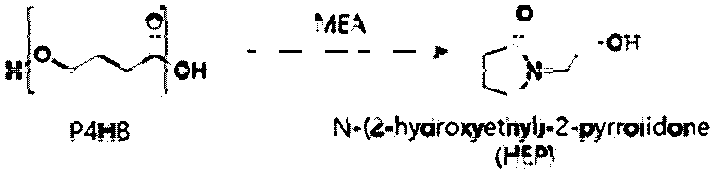

10 g of the reactant having a P4HB content of 78.3% by weight, 6.1 g of monoethanolamine (MEA), and 10 g of distilled water were charged to the reaction chamber of a batch-type, high-temperature, and high-pressure reactor. Subsequently, after purging with N₂ gas and performing a leak check, the reaction was carried out at 1 bar and 250°C for 3 hours while stirring at a speed of 500 rpm. Once the temperature in the reaction chamber had reached 250°C, the pressure during the reaction (P₂) about 30 minutes later was confirmed to be 23 bar. Upon completion of the reaction, when the temperature of the reaction chamber dropped to room temperature, the reaction liquid was recovered. Subsequently, the recovered reaction liquid was filtered through a syringe filter to remove biochar derived from cells, thereby obtaining an HEP-containing product.

### [Example 2]

Distilled water was added to the reactant containing P4HB used in Example 1 to prepare a reactant having a moisture content of 50% by weight, and an HEP-containing product was obtained through the same procedure as in Example 1.

### [Examples 3 to 10]

HEP-containing products were each obtained through the same procedure as in Example 1, except that the MEA equivalent was adjusted, the moisture content of the reactant was adjusted through the addition of distilled water as in Example 2, or the initial reaction pressure (P₁) was adjusted through injection of N₂ gas, as shown in Table 1 below.

### [Test Example 1]

The HEP-containing products obtained in Examples 1 to 10 were each analyzed by high-performance liquid chromatography (HPLC) and gas chromatography (GC), and the results are shown in Table 1 below.

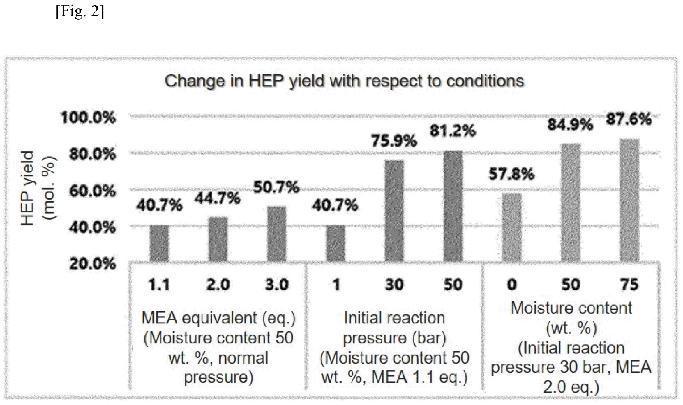

Meanwhile, the relationship between HEP yield and MEA equivalent, initial reaction pressure, and moisture content was organized in a graph, which is shown in Fig. 2.

**[Table 1]**

| | MEA equiva lent (eq.) | Moisture content (wt%) | Initial reaction pressure (P₁, bar) | Pressure during reaction (P₂, bar) | Reac tion time (hr) | Reacti on temp. (°C) | Yield (%) | | | | | | |
|---|---|---|---|---|---|---|---|---|---|---|---|---|---|
| | | | | | | | HEP | GBL | 2-Py | NMP | NVP | 4HB | 4HEBA |
| Ex. 1 | 1.1 | - | 1 | 23 | 3 | 250 | 54.6 | 1.5 | 5.4 | 0.6 | 0.0 | 4.0 | 3.2 |
| Ex. 2 | 1.1 | 50 | 1 | 25 | 3 | 250 | 40.7 | 2.7 | 8.3 | 0.9 | 0.1 | 2.2 | 7.9 |
| Ex. 3 | 1.1 | 50 | 30 | 85 | 3 | 250 | 75.9 | 2.6 | 0.3 | 0.3 | 0.1 | 0.0 | 2.4 |
| Ex. 4 | 1.1 | 50 | 50 | 109 | 3 | 250 | 81.2 | 1.4 | 0.3 | 0.3 | 0.0 | 0.0 | 1.7 |
| Ex. 5 | 2.0 | - | 1 | 28 | 3 | 250 | 60.2 | 1.5 | 3.1 | 0.2 | 0.0 | 18.7 | 16.4 |
| Ex. 6 | 2.0 | - | 30 | 79 | 3 | 250 | 57.8 | 1.9 | 0.2 | 0.2 | 0.0 | 0.0 | 0.0 |
| Ex. 7 | 2.0 | 50 | 1 | 25 | 3 | 250 | 44.7 | 6.1 | 4.4 | 0.3 | 0.0 | 25.2 | 19.1 |
| Ex. 8 | 2.0 | 50 | 30 | 80 | 3 | 250 | 84.9 | 0.8 | 0.1 | 0.0 | 0.0 | 0.0 | 0.0 |
| Ex. 9 | 2.0 | 75 | 30 | 80 | 3 | 250 | 87.6 | 0.7 | 0.1 | 0.1 | 0.0 | 0.0 | 0.7 |
| Ex. 10 | 3.0 | 50 | 1 | 23 | 3 | 250 | 50.7 | 2.9 | 3.1 | 0.1 | 0.0 | 11.0 | 9.9 |
| MEA: Monoethanolamine | | | | | | | | | | | | | |
| HEP: N-(2-hydroxyethyl)-2-pyrrolidone | | | | | | | | | | | | | |
| NMP: N-Methylpyrrolidinone | | | | | | | | | | | | | |
| NVP: N-Vinyl-2-pyrrolidone | | | | | | | | | | | | | |
| 4HB: 4-Hydroxybutyric acid | | | | | | | | | | | | | |
| GBL: γ-butyrolactone | | | | | | | | | | | | | |
| 4HEBA: 4-hydroxy-N-(2-hydroxyethyl)butanamide | | | | | | | | | | | | | |
| 2-Py: 2-Pyrrolidone | | | | | | | | | | | | | |

Referring to Table 1 above, when an HEP-containing product was prepared at a reaction temperature exceeding 200°C as in the present disclosure, an HEP-containing product with a high content of HEP, which is the intermediate desired in the present disclosure, was obtained.

In addition, referring to Fig. 2, the HEP yield was enhanced by adjusting the initial reaction pressure (P₁) and moisture content (water content) as in the present disclosure. That is, it is understood that the adjustment of the MEA equivalent did not have a significant impact on the change in HEP yield, whereas the adjustment of the initial reaction pressure (P₁) and moisture content had a significant impact on the change in HEP yield. Specifically, when the evaluation results of the HEP yield with respect to the change in moisture content of the reactant containing P4HB are taken into consideration, the HEP yield increased dramatically at 50 to 75% by weight, as compared with 0% by weight, of a moisture content when the initial reaction pressure (P₁) was 30 bar (see Examples 6, 8, and 9). In addition, as the initial reaction pressure (P₁) or pressure during the reaction (P₂) increased, the HEP yield increased significantly.

### [Comparative Example 1]

A reactant containing P4HB was prepared through the same procedure as in Example 1.

Next, 0.2 g of the reactant having a P4HB content of 78.3% by weight, 0.122 g of monoethanolamine (MEA), and 0.2 g of distilled water were charged to a 48-mL screw cap-sealed tube, and a mixture was prepared by stirring them at a speed of 300 rpm. Subsequently, the prepared mixture was reacted at 1 bar and 120°C for 3 hours using a heating stirrer. Upon completion of the reaction, the reaction liquid was recovered, and the recovered reaction liquid was filtered through a syringe filter to remove biochar derived from cells, thereby obtaining a product.

### [Comparative Examples 2 to 5]

Products were each obtained through the same procedure as in Comparative Example 1, except that the MEA equivalent was adjusted, or the moisture content of the reactant was adjusted through the addition of distilled water as in Example 2, as shown in Table 2 below.

### [Test Example 2]

The products obtained in Comparative Examples 1 to 5 were each analyzed by high-performance liquid chromatography (HPLC) and gas chromatography (GC), and the results are shown in Table 2 below.

**[Table 2]**

| | MEA equiva lent (eq.) | Moisture content (wt%) | Initial reaction pressure (bar) | Reaction temp. (°C) | Reaction time (h) | Yield (%) | | | | |
|---|---|---|---|---|---|---|---|---|---|---|
| | | | | | | HEP | 4HEBA | 4HB | GBL | 2-Py |
| C. Ex. 1 | 1.1 | - | 1 | 120 | 3 | 0.0 | 33.4 | 0.0 | 0.0 | 0.0 |
| C. Ex. 2 | 2 | - | 1 | 120 | 3 | 0.0 | 68.0 | 2.0 | 0.0 | 0.0 |
| C. Ex. 3 | 2 | 50 | 1 | 120 | 3 | 0.6 | 80.5 | 0.0 | 2.4 | 0.0 |
| C. Ex. 4 | 3 | - | 1 | 120 | 3 | 0.2 | 91.2 | 1.9 | 0.0 | 0.0 |
| C. Ex. 5 | 3 | 50 | 1 | 120 | 3 | 0.5 | 93.4 | 0.0 | 0.0 | 0.0 |
| MEA: Monoethanolamine | | | | | | | | | | |
| HEP: N-(2-hydroxyethyl)-2-pyrrolidone | | | | | | | | | | |
| 4HB: 4-Hydroxybutyric acid | | | | | | | | | | |
| GBL: γ-butyrolactone | | | | | | | | | | |
| 4HEBA: 4-hydroxy-N-(2-hydroxyethyl)butanamide | | | | | | | | | | |
| 2-Py: 2-Pyrrolidone | | | | | | | | | | |

Referring to Table 2 above, as products were prepared at a reaction temperature of 120°C, which does not satisfy the conditions of the present disclosure, conversion to HEP hardly occurred, whereby products with a significantly low HEP content were obtained. In particular, when the MEA equivalent was 2 equivalents or more at a reaction temperature of 120°C, most of the P4HB was converted to 4HEBA, not HEP, which is the intermediate desired in the present disclosure. Thus, it is understood that a significant amount of heat was further required for conversion to HEP.

### [Comparative Examples 6 to 11]

Products were each obtained through the same procedure as in Comparative Example 1, except that the MEA equivalent was adjusted while the reaction temperature was set to 200°C, or the moisture content of the reactant was adjusted through the addition of distilled water as in Example 2, as shown in Table 3 below.

### [Test Example 3]

The products obtained in Comparative Examples 6 to 11 were each analyzed by high-performance liquid chromatography (HPLC) and gas chromatography (GC), and the results are shown in Table 3 below.

**[Table 3]**

| | MEA equivale nt (eq.) | Moisture content (wt%) | Initial reaction pressure (bar) | Reaction temp. (°C) | Reaction time (h) | Yield (%) | | | | |
|---|---|---|---|---|---|---|---|---|---|---|
| | | | | | | HEP | 4HEBA | 4HB | GBL | 2-Py |
| C. Ex. 6 | 1.1 | - | 1 | 200 | 3 | 4.8 | 59.0 | 0.0 | 8.9 | 0.0 |
| C. Ex. 7 | 2 | - | 1 | 200 | 3 | 7.4 | 80.1 | 4.3 | 0.0 | 0.0 |
| C. Ex. 8 | 2 | 50 | 1 | 200 | 3 | 31.6 | 52.6 | 0.0 | 0.0 | 0.0 |
| C. Ex. 9 | 3 | - | 1 | 200 | 3 | 26.9 | 52.0 | 0.0 | 0.0 | 0.0 |
| C. Ex. 10 | 3 | 75 | 1 | 200 | 3 | 29.3 | 63.2 | 0.0 | 0.0 | 0.0 |
| C. Ex. 11 | 3 | 83 | 1 | 200 | 3 | 5.1 | 63.4 | 0.0 | 5.1 | 0.0 |
| MEA: Monoethanolamine | | | | | | | | | | |
| HEP: N-(2-hydroxyethyl)-2-pyrrolidone | | | | | | | | | | |
| 4HB: 4-Hydroxybutyric acid | | | | | | | | | | |
| GBL: γ-butyrolactone | | | | | | | | | | |
| 4HEBA: 4-hydroxy-N-(2-hydroxyethyl)butanamide | | | | | | | | | | |
| 2-Py: 2-Pyrrolidone | | | | | | | | | | |

Referring to Table 3 above, even when the reaction temperature was raised to 200°C, the amount of heat for converting P4HB to HEP was insufficient, whereby products with a low HEP content were obtained. In addition, when the moisture content of the reactant containing P4HB exceeded 50% by weight, the yield of 4HEBA and other by-products, not HEP, tended to increase.

## Claims

1. A process for preparing an N-vinyl-2-pyrrolidone intermediate, which comprises:
(1) preparing a reactant containing polyhydroxyalkanoate obtained through microbial cultivation; and
(2) reacting the reactant containing the polyhydroxyalkanoate with an amine compound at a temperature exceeding 200°C to produce an intermediate-containing product.

2. The process for preparing an N-vinyl-2-pyrrolidone intermediate according to claim 1, wherein, in step (1), the polyhydroxyalkanoate comprises a repeat unit derived from 4-hydroxybutyrate.

3. The process for preparing an N-vinyl-2-pyrrolidone intermediate according to claim 1, wherein, in step (1), the content of the polyhydroxyalkanoate contained in the reactant is 60% by weight to 90% by weight based on the total weight of the reactant.

4. The process for preparing an N-vinyl-2-pyrrolidone intermediate according to claim 1, wherein, in step (1), the moisture content of the reactant is 0% by weight to 80% by weight based on the total weight of the reactant.

5. The process for preparing an N-vinyl-2-pyrrolidone intermediate according to claim 1, wherein, in step (1), the reactant further comprises an inorganic substance.

6. The process for preparing an N-vinyl-2-pyrrolidone intermediate according to claim 5, wherein the content of the inorganic substance is greater than 0% by weight to 10% by weight based on the total weight of the reactant.

7. The process for preparing an N-vinyl-2-pyrrolidone intermediate according to claim 5, wherein the inorganic substance comprises at least one selected from the group consisting of calcium carbonate (CaCO₃), ferric sulfate (Fe₂(SO₄)₃), and sodium hydrogen carbonate (NaHCO₃).

8. The process for preparing an N-vinyl-2-pyrrolidone intermediate according to claim 1, wherein, in step (2), the initial reaction pressure of the reactant and the amine compound is 1 bar to 120 bar.

9. The process for preparing an N-vinyl-2-pyrrolidone intermediate according to claim 1, wherein, in step (2), the amine compound comprises a compound selected from the group consisting of monoethanolamine, an aqueous monoethanolamine solution, ammonia, aqueous ammonia, urea, and aqueous urea.

10. The process for preparing an N-vinyl-2-pyrrolidone intermediate according to claim 1, wherein, in step (2), the reactant and the amine compound react at an equivalent ratio of 1:1.1 to 3.

11. The process for preparing an N-vinyl-2-pyrrolidone intermediate according to claim 1, wherein, in step (2), the intermediate is N-(2-hydroxyethyl)-2-pyrrolidone.

12. An N-vinyl-2-pyrrolidone intermediate, which is prepared by the preparation process of claim 1.

13. N-vinyl-2-pyrrolidone, which is prepared from the N-vinyl-2-pyrrolidone intermediate of claim 12.

14. A process for preparing N-vinyl-2-pyrrolidone, which comprises: reacting an N-vinyl-2-pyrrolidone intermediate prepared by the preparation process of claim 1 with acetic anhydride to obtain an acetic ester intermediate; and deacetylating the obtained acetic ester intermediate.

15. A process for preparing N-vinyl-2-pyrrolidone, which comprises: subjecting an N-vinyl-2-pyrrolidone intermediate prepared by the preparation process of claim 1 to intramolecular dehydration in a vapor phase in the presence of a catalyst.
